# EUROPEAN PATENT SPECIFICATION

(11) **EP 3 811 913 B1**
(45) Date of publication and mention of the grant of the patent: **29.07.2026**
(21) Application number: 20199916.6
(22) Date of filing: 02.10.2020
(51) Int. Cl.: A61F 9/00, A62B 18/02

(54) **FACIAL RESPIRATOR AND RESPIRATORY APPARATUS**
GESICHTSATEMGERÄT UND ATEMGERÄT
RESPIRATEUR FACIAL ET APPAREIL RESPIRATOIRE

(30) Priority: 25.10.2019 GB 201915490
(43) Date of publication of application: 28.04.2021
(73) Proprietor: World Wide Welding Limited, Lutterworth LE17 4AT (GB)
(72) Inventor: Chew, Phil, Chorley, Lancashire PR6 7DN (GB)
(74) Representative: Harner-Foreman, Frank Jakob

(56) References cited:
- EP-A1- 1 266 674
- EP-A2- 1 982 740
- EP-B1- 1 266 674
- WO-A1-2013/082650
- DE-A1- 4 202 025
- DE-C2- 4 202 025
- GB-A- 2 260 084
- US-A1- 2014 373 846
- US-A1- 2016 271 355

## Description

The invention relates to a facial respirator for use in welding or grinding, and a respiratory apparatus for use in welding or grinding.

During welding/grinding, a welder/grinder risks being exposed to hazardous particulates that could lead to various forms of illness and disease, such as cancer and respiratory-related issues. To mitigate such exposure, health and safety regulations recommend a welder/grinder to be provided with respiratory equipment in order to protect the welder/grinder from unclean air containing dust, mist, metal fumes and other unsafe particulates.

The document US2014/373846 discloses a half-mask facial respirator according to the preamble of claim 1.

According to a first aspect of the invention, there is provided a half-mask facial respirator for use in welding or grinding according to claim 1, the facial respirator comprising:
a face shield for covering at least part of a user's face, the face shield including at least one port that is operably connectable to an air supply apparatus; and
a sealing member arranged on or in the face shield, the sealing member configured to be sealingly engageable with the user's face so that the face shield defines an enclosed breathing zone that is air sealed from the surrounding environment.

The provision of the or each port and the sealing member in the facial respirator of the invention enables the configuration of the face shield to reliably provide the user with a safe enclosed breathing zone that can be supplied with clean air by an air supply apparatus or a powered air-purifying respirator (PAPR). Thus, the combination of the protective eyewear, face shield and sealing member of the facial respirator of the invention provides an all-in-one solution that not only physically shields the user's face from hazardous elements such as airborne objects, molten metal and light radiation but also protects the user from breathing in contaminated air during a welding/grinding process.

In contrast, whilst conventional face shields are designed to shield the user's face from harmful ultraviolet rays and general work-related safety issues, conventional face shields do not protect the user from breathing in contaminated air. Consequently the user is either at risk of breathing in contaminated air during a welding/grinding process or is required to use additional respiratory equipment alongside a conventional face shield. The latter not only adds cost to the welding/grinding process but also increases the size and weight of equipment worn by the user which creates discomfort and restricts the user's movement.

The facial respirator of the invention therefore provides a solution that not only satisfies health and safety recommendations for welding or grinding but also provides a solution that is more comfortable, flexible and convenient for the user when compared to a conventional face shield or respiratory system.

Depending on the shape and dimensions of the face shield, the sealing member may comprise any number of sealing portions configured to be sealingly engageable with the user's face.

The sealing member may consist of a single sealing portion configured to be sealingly engageable with the user's face. The provision of a single sealing potion simplifies the configuration of the sealing member to provide the required airtight sealing engagement with the user's face, thereby reducing the complexity of manufacturing the facial respirator of the invention.

Alternatively, the sealing member may comprise a plurality of sealing portions, each of which may be configured to be sealingly engageable with a respective part of the user's face. The provision of a plurality of sealing portions provides greater flexibility in optimising the configuration of the sealing member to provide the required airtight sealing engagement with the user's face, thereby enabling the facial respirator of the invention to be used with a wide range of face shield designs.

The facial respirator may be a half-mask. As is known in the art, half-masks are configured to cover a user's mouth and nose to define an enclosed breathing zone. The breathing zone does, however, not cover a user's eyes. The user's eyes are typically not covered by the half-mask, but rather by separate, protective eyewear, or a visor.

The sealing member may be integrally formed with the face shield.

The sealing member may comprise an internally inverted portion extending from a proximal portion of the face shield.

The sealing member may be thinner than the face shield.

The sealing member may be made out of a resilient material for conforming to one or more parts of the user's face.

The sealing member may be arranged on or in the face shield in various ways to configure the sealing member to sealingly engage various parts of the user's face.

In embodiments of the invention, the sealing member may be arranged along at least one edge of the face shield. Arranging the sealing member along at least one edge of the face shield allows the face shield to define a larger enclosed breathing zone so as to improve the breathing capacity of the facial respirator of the invention.

It will be appreciated that the sealing member may be arranged along one or more parts of the face shield that correspond to one or more parts of the user's face, non-limiting examples of which are described as follows.

In a first example, the sealing member may be arranged along cheek sections of the face shield so as to be sealingly engageable with the user's cheeks.

In a second example, the sealing member may be arranged along a chin section of the face shield so as to be sealingly engageable with the user's chin.

In further embodiments of the invention, the facial respirator may include an elastic member configured for holding the sealing member in sealing engagement with the user's face. In such embodiments, the elastic member may be a band, strap or cord, and/or a length of the elastic member may be adjustable.

The provision of the elastic member not only enables the sealing member to be held in sealing engagement with different face shapes and dimensions but also improves the seal formed between the sealing member and the user's face.

In another embodiment, the at least one port that is operably connectable to an air supply apparatus is an air inlet port, wherein the face shield comprises a first air outlet port and two cheek sections adapted to cover a user's cheeks, when in use, the air inlet port being arranged at a first cheek section of the face shield, the first air outlet port being arranged at a second cheek section of the face shield, and the first and second cheek sections of the face shield being connected by a nose section of the face shield adapted to cover a user's nose, when in use.

The face shield comprises a second air outlet port arranged at a chin section adapted to cover a user's cheek, when in use.

The facial respirator comprises an angled air inlet connector piece, wherein the angled air inlet connector piece comprises a first end for connection to a hose of an air supply apparatus, when in use, and a second end rotatably attached to the at least one port that is operably connectable to an air supply apparatus, and wherein the first end extends in a substantially perpendicular direction to the second end.

The sealing member may be secured to the face shield in a variety of ways, non-limiting examples of which are described as follows.

In a first example, the facial respirator may include a retainer configured to secure the sealing member to the face shield. Optionally the retainer may be fastened to the face shield using at least one screw fastener and/or at least one spring clip, and/or the retainer may be made out of a plastic material.

In a second example, the facial respirator may include at least one hook-and-loop fastener configured to secure the sealing member to the face shield.

In a third example, the facial respirator may include at least one nut and bolt fastening arrangement configured to secure the sealing member to the face shield.

In a preferred embodiment of the invention, the sealing member is made out of a resilient material for conforming to one or more parts of the user's face. Using a resilient material capable of conforming to one or more parts of the user's face enhances the airtight sealing engagement between the sealing member and the user's face, thus improving the ability of the face shield to provide a safe enclosed breathing zone.

The sealing member may be made out of, but is not limited to, a flame retardant material (such as flame retardant cotton) and/or a synthetic sealant material.

The protective eyewear may be any type of protective eyewear that is suitable for use in welding/grinding. For example, the protective eyewear may be a pair of goggles.

Optionally the protective eyewear may include a visual filter. This improves the ability of the protective eyewear to protect the eyes of the user from high levels of light radiation. Preferably the protective eyewear includes an automatic darkening filter.

The protective eyewear and face shield of the invention are designed to comply to the respective safety norms for welding or grinding. The combination of the enclosed breathing zone and the faceshield/eyewear in the facial respirator of the invention provides a compact and lightweight solution that can be comfortably worn by the user while accessing tight spaces, such as an underside of a vehicle. This is because the combination of the enclosed breathing zone and the facial respirator of the invention removes the need for additional bulky respiratory equipment and/or filtering equipment that would hinder the user from accessing tight spaces. For example, the large size of a helmet/hood/visor restricts the user from accessing aces that are too small to accommodate the helmet/hood/visor.

According to a second aspect of the invention, there is provided a respiratory apparatus for use in welding or grinding, the respiratory apparatus comprising the facial respirator according to any one of the embodiments of the first aspect of the invention described hereinabove and an air supply apparatus, wherein the or each port of the face shield is operably connected to the air supply apparatus.

The features and advantages of the facial respirator of the first aspect of the invention apply mutatis mutandis to the respiratory apparatus of the second aspect of the invention.

In embodiments of the invention, the air supply apparatus may be a powered air apparatus or a supplied air apparatus.

In further embodiments of the invention, the air supply apparatus may be configurable to set an air pressure inside the enclosed breathing zone to be higher than an air pressure of the surrounding environment. This creates a positive pressure inside the enclosed breathing zone that limits ingress of contaminated air from the surrounding environment in the event of a leak in the facial respirator.

In still further embodiments of the invention, the respiratory apparatus may include an air conduit configured to connect the or each port of the face shield to the air supply apparatus. The air conduit may be, for example, a flexible hose.

According to another aspect, there is provided a personal protection assembly comprising:
a face mask, helmet, or hood including a visor; and
any of the above facial respirators, wherein the facial respirator is adapted to be arranged between the face mask, helmet, or hood and the user's face.

A preferred embodiment of the invention will now be described, by way of a non-limiting example, with reference to the accompanying drawings in which:
Figure 1 shows a facial respirator according to a first embodiment of the invention;
Figure 2 shows a side view of the facial respirator of Figure 1;
Figures 3A to 3D show various view of a facial respirator according to a second embodiment of the invention; and
Figure 4 shows a personal protection assembly according to an embodiment of the present invention.

The figures are not necessarily to scale, and certain features and certain views of the figures may be shown exaggerated in scale or in schematic form in the interests of clarity and conciseness.

The following embodiment of the invention is described with reference to a welding respiratory apparatus for use in welding, but it will be appreciated that the following embodiment of the invention applies mutatis mutandis to a grinding respiratory apparatus for use in grinding.

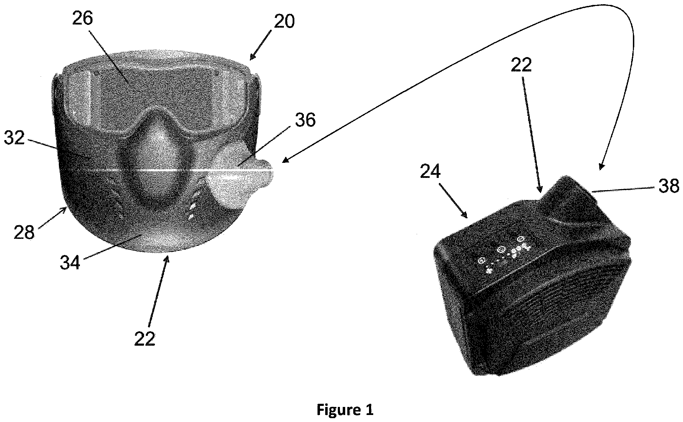

A facial respirator according to an embodiment of the invention is shown in Figure 1 and is designated generally by the reference numeral 20.

The facial respirator 20 forms part of a welding respiratory apparatus 22, which also comprises an air conduit (not shown) and an air supply apparatus 24.

The facial respirator 20 comprises a protective eyewear 26, a face shield 28 and a sealing member 30.

The protective eyewear 26 is in the form of a pair of goggles 26, which in other embodiments may be replaced by any other type of protective eyewear that is suitable for use in welding. The pair of goggles 26 includes an automatic darkening filter that turns dark the moment a welding arc is formed and turns transparent when the welding arc dissipates. This provides a welder wearing the goggles 26 with automatic protection from high levels of light radiation without having to manually turn the filter on and off.

It is envisaged that, in other embodiments of the invention, the protective eyewear may utilise a different type of visual filter or may omit the visual filter.

The face shield 28 is configured for covering a welder's nose, mouth, cheeks and chin. The face shield 28 is fixed to an underside of the goggles 26 so that, in use, the face shield 28 and googles 26 provides the welder with full facial protection during a welding process.

The sealing member 30 consists of a single unitary sealing portion that is mounted along the edges of the cavity of the face shield 28 that correspond to cheek sections 32 and a chin section 34 of the face shield 28. This is so that, when the face shield 28 is worn by the welder, the sealing member 30 engages the welder's cheeks and chin so as to form a seal between the face shield 28 and the welder's cheeks and chin. In this way the face shield 28 defines an enclosed breathing zone that is air sealed from the surrounding environment, where the sealing member 30 prevents ingress of air into the enclosed breathing zone from the surrounding environment.

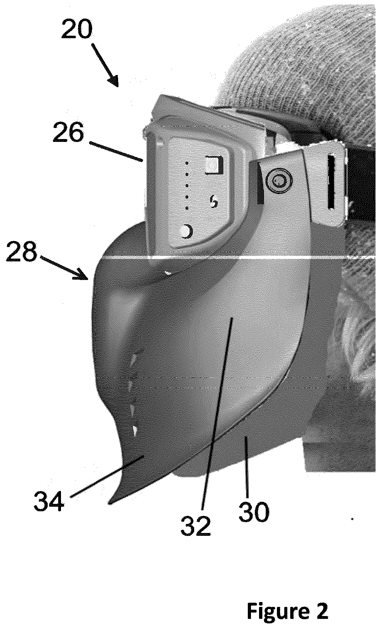

Figure 2 shows a side view of the facial respirator 20 where the sealing member engages the welder's cheeks and chin. A port 36 formed on a wall of the face shield 28 is graphically omitted from Figure 2 for the purposes of illustrating the sealing member 30.

Different exemplary ways of securing the sealing member 30 to the face shield 28 includes use of:
- a plastic retainer to secure the sealing member 30 to the face shield 28. The retainer may be fastened to the face shield 28 using at least one screw fastener and/or at least one spring clip;
- at least one hook-and-loop fastener to secure the sealing member 30 to the face shield 28; and/or
- at least one nut and bolt fastening arrangement to secure the sealing member 30 to the face shield 28.

The facial respirator 20 includes an elastic member that in use holds the sealing member 30 in sealing engagement with the welder's cheeks and chin. The provision of the elastic member not only enables the sealing member 30 to be held in sealing engagement with different face shapes and dimensions but also improves the seal formed between the sealing member 30 and the welder's cheeks and chin. The elastic member may take the form of a band, strap or pull cord. A length of the elastic member may be adjustable.

By making the sealing member 30 out of a resilient material, the sealing member 30 is able to conform to the welder's cheeks and chin in order to not only enhance the airtight seal between the face shield 28 and the welder's cheeks and chin but also enable the facial respirator 20 to be used by different people having facial features of different shapes and dimensions. Optionally the sealing member 30 may be made out of a flame retardant material (such as flame retardant cotton) and/or a synthetic sealant material.

Depending on the shape and dimensions of the face shield, the sealing member may comprise any number of sealing portions configured to be sealingly engageable with the welder's face. For example, the sealing member may comprise a plurality of sealing portions, each of which may be configured to be sealingly engageable with a respective part of the welder's face. Using multiple sealing portions provides greater flexibility in optimising the configuration of the sealing member to form the airtight seal between the face shield and the welder's face and thereby enables the facial respirator to be used with a wide range of face shield designs.

The air flow conduit is in the form of a flexible hose. One end of the flexible hose is sealingly connected to the port 36 formed on a wall of the face shield 28 so that the flexible hose is in air communication with a cavity of the face shield 28. The cavity defines a breathing zone around the nose and mouth of the welder wearing the face shield 28.

The air supply apparatus 24 is in the form of a powered air supply unit 24 that is configured for supplying air to the facial respirator 20. More particularly, the powered air supply unit 24 comprises a port 38 that is sealingly connected to the other end of the flexible hose, a fan for blowing air out of the port 38, a filter arranged to filter the air before it exits the port 38, a motor for driving the fan, and a controller for controlling the motor and thereby controlling the fan. The controller is configured to receive input commands to start the motor and to change the motor speed and thereby change the fan speed. Input commands may be inputted via a control panel on the powered air supply unit 24. The powered air supply unit 24 includes a portable power source, such as a battery, so that the welder is not tethered to a fixed location when using the powered air supply unit.

In other embodiments of the invention, the power air supply unit may be replaced by a supplied air system, such as a compressed air line.

Interconnecting the face shield 28 and the powered air supply unit 24 via the flexible hose enables the operation of the powered air supply unit 24 to deliver filtered air to the enclosed breathing zone defined by the face shield 28. In a preferred mode of operation of the powered air supply unit 24, the powered air supply unit 24 is configured to set an air pressure inside the enclosed breathing zone to be higher than an air pressure of the surrounding environment, which is typically 1 atm at sea level. This creates a positive pressure inside the enclosed breathing zone that limits ingress of contaminated air from the surrounding environment during a welding process in the event of a leak in the facial respirator 20.

In the embodiment shown in Figures 1 and 2, the goggles 26 and face shield 28 define separate enclosed zones covering the welder's face. It will however be appreciated that, in other embodiments of the invention, the goggles and face shield may define a unitary enclosed zone covering the welder's face.

Therefore, by providing the port 36 in the face shield 28 and arranging the sealing member 30 between the face shield 28 and the welder's face, the facial respirator 20 is configured to provide the welder with a safe enclosed breathing zone that can be supplied with clean air by the powered air supply unit 24. The combination of the goggles 26, face shield 28 and sealing member 30 of the facial respirator 20 provides an all-in-one solution that physically shields the welder's face from hazardous elements and protects the welder from breathing in contaminated air during a welding process.

This is in contrast to conventional face shields that are incapable of adequately protecting the welder from breathing in contaminated air during a welding process. In fact the inventor has found that the facial respirator 20 of the invention is capable of providing respiratory protection that conforms to EN12491, which is not achievable by conventional face shield and goggles combinations. When using conventional face shield and goggles combinations, the welder is either at risk of breathing in contaminated air during a welding process or is required to use additional respiratory equipment alongside the conventional face shield. The latter not only adds cost to the welding process but also increases the size and weight of equipment worn by the welder which creates discomfort and restricts the welder's movement.

The facial respirator 20 of the invention therefore provides a solution that not only satisfies health and safety regulations recommendations for welding but also is more cost-effective and convenient for the welder when compared to a conventional face shield.

In addition, the combination of the enclosed breathing zone and the automatic darkening filter results in a compact and lightweight facial respirator 20 that enables the welder wearing the facial respirator 20 to access tight spaces, such as an underside of a car, which is not accessible while using a welding helmet/hood/visor and additional respiratory equipment.

It is envisaged that, in other embodiments of the invention, the face shield may include multiple ports formed on a wall thereon, each port being operably connectable to the air supply apparatus.

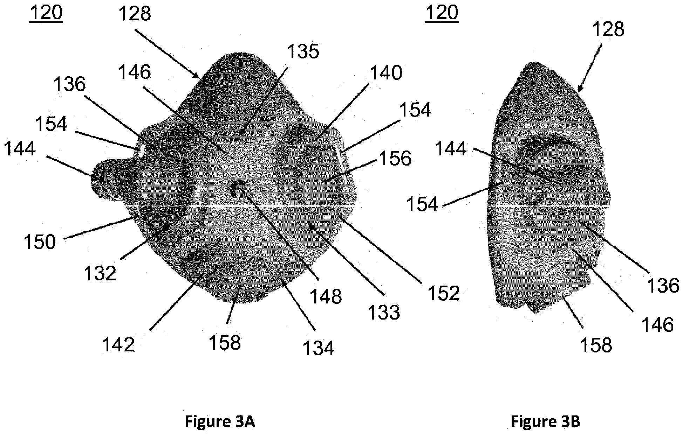

Figures 3A to 3D show another embodiment of a facial respirator 120 according to the present disclosure. The facial respirator 120 is a half-mask facial respirator, which is configured to surround the operator's mouth and nose to define a breathing zone. The half-mask facial respirator 120 does not cover the operator's eyes.

The half-mask facial respirator 120 shown in Figures 3A to 3D comprises a face shield 128. The face shield 128 comprises a plurality of air inlet and outlet ports. In particular, the face shield 128 comprises an air inlet port 136 and two air outlet ports 140, 142.

The face shield 128 comprises two cheek sections adapted to cover a user's cheeks, when in use. A first cheek section 132 is arranged opposite a second cheek section 133. The first cheek section 132 is configured to cover the operator's right cheek, when in use. The second cheek section 133 is configured to cover the operator's left cheek, when in use. The face shield 128 further comprises a chin section 134 configured to cover a users' chin, when in use. The first and second cheek sections 132, 133 are connected by a nose section 135, which is configured to cover an operator's nose when in use.

The air inlet port 136 of the embodiment shown in Figures 3A to 3D is arranged at the first cheek section 132. A first outlet port 140 is arranged at the second cheek section 133. A second air outlet port 142 is arranged at the chin section 134.

The facial respirator 120 comprises an angled air inlet connector piece 144, which will be described in more detail below. The angled air inlet connector piece 144 is connected to the air inlet port 136.

The facial respirator 120 includes a strap retainer 146. The strap retainer 146 is removably connected to the face shield 128. In particular, the strap retainer 146 is removably connected to the nose section 135, particularly to a spigot 148 protruding from the nose section 135. The strap retainer 148 includes a first opening 150 and a second opening 152. The first opening 150 surrounds the air inlet port 136. The second opening 152 surrounds the first outlet port 140. In other words, the strap retainer 146 is substantially goggle shaped with two openings 150, 152 surrounding the air inlet and outlet ports 136, 140 arranged on the first and second cheek sections 132, 133.

The strap retainer 146 includes eyelets 154 on both ends of the strap retainer 146. The eyelets 154 are configured to receive ends of a corresponding, flexible strap that may be used to secure the face shield 128 against a user's face, when in use. The first air outlet port 140 is provided with a first outlet valve 156. The second outlet port 142 is provided with a second air outlet valve 158. The first and second air outlet valves 156, 158 may be one-way valves that allow air to escape from the enclosed breathing zone defined by the face shield 128.

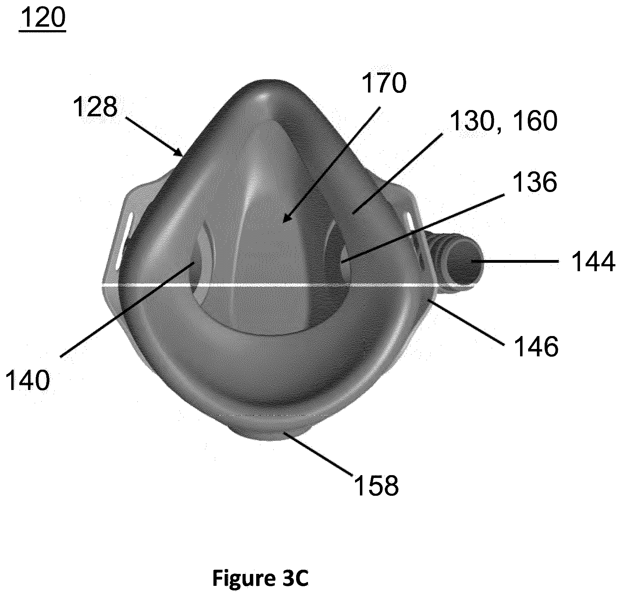

Referring to Figure 3C, there is shown a perspective rear view of the facial respirator 120 shown in Figure 3A. The rear view shown in Figure 3C shows a proximal end of the face shield 128, i.e. an end that is in contact with the user's face, when in use. A sealing member 130 is arranged at the proximal end of the face shield 128 of the facial respirator 120. IN the embodiment of Figures 3A to 3D, the sealing member 130 is integrally formed with the face shield 128. Accordingly, the sealing member 130 and the face shield 128 are made from the same material.

The sealing member 130 is made from a resilient material and thus sufficiently flexible to conform to one or more parts of the user's face, particularly the user's chin, cheeks, and nasal bridge. By conforming to the user's face, the sealing member 130 prevents air from being introduced into the breathing zone of the facial respirator 120 between the user's face and the face shield 128. In other words, the sealing member 130 facilitates a gap-free fit of the facial respirator 120 on the user's face.

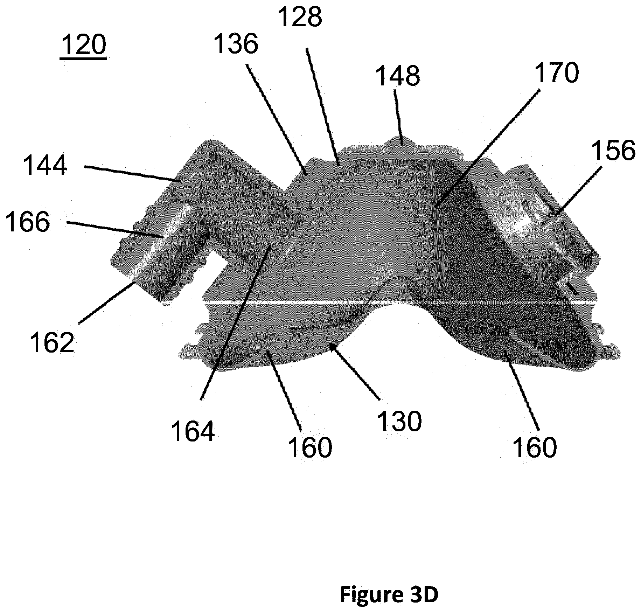

The sealing member 130 of the facial respirator 120 according to the embodiment shown in Figures 3A to 3D comprises an internally inverted portion 160 extending from the proximal portion of the face shield 128. The internally inverted portion 160 is best shown in Figure 3D, which shows a cross section through the facial respirator 120 shown in Figures 3A to 3C. The inverted portion 160 is a thin-walled portion of the facial respirator 120 that is folded over into the breathing zone 170 defined by the face shield 128.

The sealing member 130, and particularly the internally inverted portion 160 of the sealing member 130, is thinner than the face shield 128. In particular, a wall thickness of the sealing member 130 is thinner than an average wall thickness of the various portions (chin, nose, cheek portions) of the face shield 128. Accordingly, the sealing member 130 is more flexible than the face shield 128, such that the sealing member 130 may deform when the facial respirator 120 is strapped to the user's face, whereas the face shield 128 will largely maintain its shape, which is designed to define the enclosed breathing zone discussed above. It will be appreciated that, in alternative variants, the sealing member 130 may have the same thickness as the face shield as long as the sealing member 130 is more flexible. For example, the sealing member may be made of a different, more flexible material than the face shield.

Further referring to Figure 3D, there is shown a cross section of the angled air inlet connector piece 144. The angled air inlet connector piece comprises a first end 162 for connection to a hose (not shown) of a corresponding air supply apparatus, when in use. The angled air inlet connector piece comprises a second end 164, which is rotatably attached to the air inlet port 136. In other words, the angled air inlet connector piece is freely rotatable within the air inlet port 136 about the second end 164. The angled air inlet connector piece 144 comprises an air channel 166 extending between the first and second end 162, 164. Air supplied from an air supply apparatus via a hose (not shown) that is attached to the first end 162 of the angled air inlet connector piece 144 may, therefore, be introduced into the enclosed breathing zone 170 via the air channel 166.

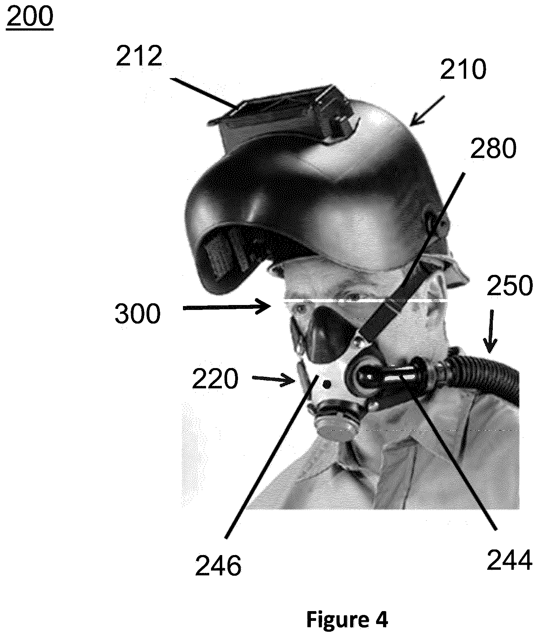

The first end 162 of the angled air inlet connector piece 144 extends in a substantially perpendicular direction to the second end 164. Accordingly, a hose of an air supply apparatus does not need to be attached directly to the air inlet port 136 and, therefore, perpendicularly to an outer surface of the face shield 128. Rather, the hose of a corresponding air supply apparatus may be attached to the first end 162 of the angled air inlet connector piece 144 in a manner that is substantially parallel with an outer surface of the face shield 128. This is particularly space saving and enables the facial respirator of this embodiment to be used together with a variety of face masks, helmets, and/or hoods including a visor, as will be described in more detail with reference to Figure 4 below. It should be understood that the angled air inlet connector piece 162 does not need to include a 90 degree angle as shown in Figure 3d. Rather, it is important that the first and second ends 162, 164 extend in substantially perpendicular directions, such that the hose maybe connected in parallel with the face shield 128. In some embodiments, the angled air inlet connector piece may be a tube that is radiused between its first and second end.

Turning to Figure 4, there is shown a schematic representation of a personal protection assembly 200 in accordance with the present disclosure. The personal protection assembly 200 shown in Figure 4 comprises a welding mask 210 and a facial respirator 220, substantially identical to the facial respirator 120 explained above. The welding mask 210 comprises a visor 212, which is employed to protect the user from heat and radiation caused by the welding process, whilst, at the same time, allowing the user to see their workstation.

As mentioned before with respect to the protective eye wear, the visor 212 may include an auto-darkening filter. As illustrated in Figure 4, the welding mask 210 may substantially completely cover a user's face 300. However, in other embodiments, the personal protection assembly may include other protective devices, such as welding helmet, which fully covers the user's head, or surgical hoods, which are typically constructed of loose material. The welding mask 210 may be attached to the user's head 300 by means of a headgear.

In order to be light weight and comfortable to wear, welding masks and other protective face coverings typically conform closely to the user's head 300. Accordingly, such protective face coverings typically provide little free space between the protective face covering and a user's head. Accordingly, standard facial respirators cannot be worn under protective face covers, particularly due the arrangement of their air inlet and outlet ports.

The facial respirators 20, 120, 220 of the present disclosure are different in that the air inlet port is arranged at one of the cheek portions of the face shield, as described above. Furthermore, an angled air inlet connector piece 244 may be provided, which facilitates space saving attachment of the hose 250 to the air inlet port of the face shield.

The arrangement of at least one air outlet port at the chin section of the face shield prevents the visor 212 from steaming up.

The facial respirator 220 shown in Figure 4 is attached to a head gear of the welding mask by means of a resilient strap 280. However, it is equivalently feasible to attach the facial respirators of the present disclosure to the back of the user's head 300 via one or more resilient straps. The resilient strap 280 is attached to a strap retainer 246 via corresponding eyelets.

The personal protection assembly 200 shown in Figure 4 may be used with most available protective face coverings, such as welding masks, welding helmets, and/or surgical hoods, due to the space saving construction of the facial respirator discussed above.

## Claims

1. A half-mask facial respirator (120) for use in welding or grinding, the facial respirator (120) comprising:
a face shield (128) for covering at least part of a user's face, the face shield (128) including at least one port that is operably connectable to an air supply apparatus; and
a sealing member (130) arranged on or in the face shield (128), the sealing member (130) configured to be sealingly engageable with the user's face so that the face shield (128) defines an enclosed breathing zone that is air sealed from the surrounding environment,
wherein the at least one port that is operably connectable to an air supply apparatus is an air inlet port (136), wherein the face shield (128) comprises a first air outlet port and two cheek sections (132, 133) adapted to cover a user's cheeks, when in use, the air inlet port (136) being arranged at a first cheek section of the face shield (128), the first air outlet port being arranged at a second cheek section of the face shield (128), and the first and second cheek sections (132, 133) of the face shield (128) being connected by a nose section of the face shield (128) adapted to cover a user's nose, when in use,
**characterized in that** the face shield (128) comprises a second air outlet port arranged at a chin section (134) adapted to cover a user's cheek, when in use,
and **in that** the facial respirator comprises an angled air inlet connector piece, wherein the angled air inlet connector piece comprises a first end for connection to a hose of an air supply apparatus, when in use, and a second end rotatably attached to the at least one port that is operably connectable to an air supply apparatus, and wherein the first end extends in a substantially perpendicular direction to the second end.

2. The half-mask facial respirator (120) according to Claim 1, wherein the sealing member (130) consists of a single sealing portion configured to be sealingly engageable with the user's face, or wherein the sealing member (130) comprises a plurality of sealing portions, each of which is configured to be sealingly engageable with a respective part of the user's face.

3. The half-mask facial respirator (120) according to Claim 1 or Claim 2, wherein the sealing member (130) is integrally formed with the face shield (128).

4. The half-mask facial respirator (120) according to Claim 3, wherein the sealing member (130) comprises an internally inverted portion extending from a proximal portion of the face shield (128).

5. The half-mask facial respirator (120) according to Claim 3 or Claim 4, wherein the sealing member (130) is thinner than the face shield (128).

6. The half-mask facial respirator (120) according to any one of the preceding claims, wherein the sealing member (130) is made out of a resilient material for conforming to one or more parts of the user's face.

7. The half-mask facial respirator (120) according to any one of the preceding claims, wherein the sealing member (130) is arranged along at least one edge of the face shield (128).

8. The half-mask facial respirator (120) according to any one of the preceding claims, wherein the sealing member (130) is arranged along cheek sections (132, 133) of the face shield (128) so as to be sealingly engageable with the user's cheeks, and/or wherein the sealing member (130) is arranged along a chin section (134) of the face shield (128) so as to be sealingly engageable with the user's chin.

9. The half-mask facial respirator (120) according to any one of the preceding claims, including an elastic member configured for holding the sealing member (130) in sealing engagement with the user's face.

10. The half-mask facial respirator (120) according to Claim 9, wherein the elastic member is a band, strap or cord, and/or wherein a length of the elastic member is adjustable.

11. A respiratory apparatus for use in welding or grinding, the respiratory apparatus comprising a half-mask facial respirator (120) according to any one of the preceding claims and an air supply apparatus, wherein the at least one port of the face shield (128) is operably connected to the air supply apparatus, and wherein the air supply apparatus is configurable to set an air pressure inside the enclosed breathing zone to be higher than an air pressure of the surrounding environment.

12. A personal protection assembly (200) comprising:
a face mask (210), helmet, or hood including a visor (212); and
a half-mask facial respirator (120) according to any one of Claims 1 to 10, wherein the half-mask facial respirator (120) is adapted to be arranged between the face mask, helmet, or hood and the user's face.

## Patentansprüche

1. Halbmasken-Atemschutzgerät (120) zur Verwendung beim Schweißen oder Schleifen, wobei das Atemschutzgerät (120) Folgendes umfasst:
einen Gesichtsschild (128) zum Abdecken mindestens eines Teils des Gesichts eines Benutzers, wobei der Gesichtsschild (128) mindestens einen Anschluss aufweist, der mit einer Luftversorgungseinrichtung wirkverbindbar ist, und
ein Dichtungsglied (130), das an oder in dem Gesichtsschild (128) angeordnet ist, wobei das Dichtungsglied (130) dazu ausgestaltet ist, in Dichteingriff mit dem Gesicht des Benutzers bringbar zu sein, so dass der Gesichtsschild (128) eine umschlossene Atemzone definiert, die gegenüber der umliegenden Umgebung luftdicht abgeschlossen ist,
wobei der mindestens eine Anschluss, der mit einer Luftversorgungseinrichtung wirkverbindbar ist, ein Lufteinlassanschluss (136) ist, wobei der Gesichtsschild (128) einen ersten Luftauslassanschluss und zwei Wangenabschnitte (132, 133) umfasst, die dazu ausgeführt sind, im Gebrauch die Wangen eines Benutzers abzudecken,
wobei der Lufteinlassanschluss (136) an einem ersten Wangenabschnitt des Gesichtsschilds (128) angeordnet ist, wobei der erste Luftauslassanschluss an einem zweiten Wangenabschnitt des Gesichtsschilds (128) angeordnet ist und der erste und der zweite Wangenabschnitt (132, 133) des Gesichtsschilds (128) durch einen Nasenabschnitt des Gesichtsschilds (128) verbunden sind, der dazu ausgeführt ist, im Gebrauch die Nase eines Benutzers abzudecken,
**dadurch gekennzeichnet, dass**
der Gesichtsschild (128) einen zweiten Luftauslassanschluss umfasst, der an einem Kinnabschnitt (134) angeordnet ist, der dazu ausgeführt ist, im Gebrauch die Wange eines Benutzers abzudecken,
und dass
das Atemschutzgerät ein abgewinkeltes Lufteinlassverbinderstück umfasst, wobei das abgewinkelte Lufteinlassverbinderstück ein erstes Ende zum Verbinden mit einem Schlauch einer Luftversorgungseinrichtung im Gebrauch und ein zweites Ende umfasst, das drehbar an dem mindestens einen Anschluss angebracht ist, der mit einer Luftversorgungseinrichtung wirkverbindbar ist, und wobei sich das erste Ende in einer im Wesentlichen senkrechten Richtung zu dem zweiten Ende erstreckt.

2. Halbmasken-Atemschutzgerät (120) nach Anspruch 1, wobei das Dichtungsglied (130) aus einem einzigen Dichtungsabschnitt besteht, der dazu ausgestaltet ist, mit dem Gesicht des Benutzers in Dichteingriff gebracht zu werden, oder wobei das Dichtungsglied (130) eine Vielzahl von Dichtungsabschnitten umfasst, die jeweils dazu ausgestaltet sind, mit einem jeweiligen Teil des Gesichts des Benutzers in Dichteingriff gebracht zu werden.

3. Halbmasken-Atemschutzgerät (120) nach Anspruch 1 oder Anspruch 2, wobei das Dichtungsglied (130) integral mit dem Gesichtsschild (128) ausgebildet ist.

4. Halbmasken-Atemschutzgerät (120) nach Anspruch 3, wobei das Dichtungsglied (130) einen innen umgekehrten Abschnitt umfasst, der sich von einem proximalen Abschnitt des Gesichtsschilds (128) erstreckt.

5. Halbmasken-Atemschutzgerät (120) nach Anspruch 3 oder Anspruch 4, wobei das Dichtungsglied (130) dünner als der Gesichtsschild (128) ist.

6. Halbmasken-Atemschutzgerät (120) nach einem der vorhergehenden Ansprüche, wobei das Dichtungsglied (130) aus einem nachgiebigen Material hergestellt ist, um sich an einen oder mehrere Teile des Gesichts des Benutzers anzupassen.

7. Halbmasken-Atemschutzgerät (120) nach einem der vorhergehenden Ansprüche, wobei das Dichtungsglied (130) entlang mindestens eines Rands des Gesichtsschilds (128) angeordnet ist.

8. Halbmasken-Atemschutzgerät (120) nach einem der vorhergehenden Ansprüche, wobei das Dichtungsglied (130) entlang Wangenabschnitten (132, 133) des Gesichtsschilds (128) angeordnet ist, um mit den Wangen des Benutzers in Dichteingriff bringbar zu sein, und/oder wobei das Dichtungsglied (130) entlang eines Kinnabschnitts (134) des Gesichtsschilds (128) angeordnet ist, um mit dem Kinn des Benutzers in Dichteingriff bringbar zu sein.

9. Halbmasken-Atemschutzgerät (120) nach einem der vorhergehenden Ansprüche, aufweisend ein elastisches Glied, das zum Halten des Dichtungsglieds (130) in Dichteingriff mit dem Gesicht des Benutzers ausgestaltet ist.

10. Halbmasken-Atemschutzgerät (120) nach Anspruch 9, wobei das elastische Glied ein Band, ein Gurt oder eine Schnur ist und/oder wobei eine Länge des elastischen Glieds verstellbar ist.

11. Atemschutzgerät zur Verwendung beim Schweißen oder Schleifen, wobei das Atemschutzgerät ein Halbmasken-Atemschutzgerät (120) nach einem der vorhergehenden Ansprüche und eine Luftversorgungseinrichtung umfasst, wobei der mindestens eine Anschluss des Gesichtsschilds (128) mit der Luftversorgungseinrichtung wirkverbunden ist und wobei die Luftversorgungseinrichtung dazu ausgestaltet werden kann, einen Luftdruck innerhalb der umschlossenen Beatmungszone so einzustellen, dass er höher als ein Luftdruck der umliegenden Umgebung ist.

12. Baugruppe (200) für den persönlichen Schutz, umfassend:
eine Gesichtsmaske (210), einen Helm oder eine Kapuze, die ein Visier (212) aufweist, und
ein Halbmasken-Atemschutzgerät (120) nach einem der Ansprüche 1 bis 10, wobei das Halbmasken-Atemschutzgerät (120) dazu ausgeführt ist, zwischen der Gesichtsmaske, dem Helm oder der Kapuze und dem Gesicht des Benutzers angeordnet zu sein.

## Revendications

1. Respirateur facial de type demi-masque (120) destiné à être utilisé lors d'opérations de soudage ou de meulage, le respirateur facial (120) comprenant :
un écran facial (128) destiné à couvrir au moins une zone du visage d'un utilisateur, l'écran facial (128) comportant au moins un orifice apte à être raccordé de manière fonctionnelle à un appareil d'alimentation en air ; et
un élément d'étanchéité (130) agencé sur ou dans l'écran facial (128), l'élément d'étanchéité (130) étant configuré pour pouvoir venir en contact d'étanchéité avec le visage de l'utilisateur de sorte que l'écran facial (128) définisse une zone respiratoire fermée qui est étanche à l'air par rapport au milieu ambiant,
l'au moins un orifice apte à être raccordé de manière fonctionnelle à un appareil d'alimentation en air étant un orifice d'entrée d'air (136), l'écran facial (128) comprenant un premier orifice de sortie d'air et deux parties de joue (132, 133) adaptées à couvrir les joues d'un utilisateur, lors de l'utilisation, l'orifice d'entrée d'air (136) étant agencé au niveau d'une première partie de joue de l'écran facial (128), le premier orifice de sortie d'air étant agencé au niveau d'une deuxième partie de joue de l'écran facial (128),
et les première et deuxième parties de joue (132, 133) de l'écran facial (128) étant reliées par une partie de nez de l'écran facial (128) adaptée à couvrir le nez d'un utilisateur, lors de l'utilisation,
**caractérisé en ce que**
l'écran facial (128) comprend un deuxième orifice de sortie d'air agencé au niveau d'une partie de menton (134) adaptée à couvrir la joue d'un utilisateur, lors de l'utilisation,
et **en ce que**
le respirateur facial comprend une pièce de raccord d'entrée d'air coudée, la pièce de raccord d'entrée d'air coudée comprenant une première extrémité destinée à être raccordée à un tuyau flexible d'un appareil d'alimentation en air, lors de l'utilisation, et une deuxième extrémité fixée de manière rotative à l'au moins un orifice apte à être raccordé de manière fonctionnelle à un appareil d'alimentation en air, et la première extrémité s'étendant dans une direction sensiblement perpendiculaire à la deuxième extrémité.

2. Respirateur facial de type demi-masque (120) selon la revendication 1, dans lequel l'élément d'étanchéité (130) se compose d'une seule portion d'étanchéité configurée pour pouvoir venir en contact d'étanchéité avec le visage de l'utilisateur, ou dans lequel l'élément d'étanchéité (130) comprend une pluralité de portions d'étanchéité, chacune étant configurée pour pouvoir venir en contact d'étanchéité avec une zone respective du visage de l'utilisateur.

3. Respirateur facial de type demi-masque (120) selon la revendication 1 ou la revendication 2, dans lequel l'élément d'étanchéité (130) est formé d'un seul tenant avec l'écran facial (128).

4. Respirateur facial de type demi-masque (120) selon la revendication 3, dans lequel l'élément d'étanchéité (130) comprend une portion inversée intérieurement s'étendant à partir d'une portion proximale de l'écran facial (128).

5. Respirateur facial de type demi-masque (120) selon la revendication 3 ou la revendication 4, dans lequel l'élément d'étanchéité (130) est plus mince que l'écran facial (128).

6. Respirateur facial de type demi-masque (120) selon l'une quelconque des revendications précédentes, dans lequel l'élément d'étanchéité (130) est constitué d'un matériau souple destiné à épouser une ou plusieurs zones du visage de l'utilisateur.

7. Respirateur facial de type demi-masque (120) selon l'une quelconque des revendications précédentes, dans lequel l'élément d'étanchéité (130) est agencé le long d'au moins un bord de l'écran facial (128).

8. Respirateur facial de type demi-masque (120) selon l'une quelconque des revendications précédentes, dans lequel l'élément d'étanchéité (130) est agencé le long de parties de joue (132, 133) de l'écran facial (128) de manière à pouvoir venir en contact d'étanchéité avec les joues de l'utilisateur, et/ou dans lequel l'élément d'étanchéité (130) est agencé le long d'une partie de menton (134) de l'écran facial (128) de manière à pouvoir venir en contact d'étanchéité avec le menton de l'utilisateur.

9. Respirateur facial de type demi-masque (120) selon l'une quelconque des revendications précédentes, comportant un élément élastique configuré pour maintenir l'élément d'étanchéité (130) en contact d'étanchéité avec le visage de l'utilisateur.

10. Respirateur facial de type demi-masque (120) selon la revendication 9, dans lequel l'élément élastique est une bande, une sangle ou un cordon, et/ou dans lequel une longueur de l'élément élastique est réglable.

11. Appareil respiratoire destiné à être utilisé lors d'opérations de soudage ou de meulage, l'appareil respiratoire comprenant un respirateur facial de type demi-masque (120) selon l'une quelconque des revendications précédentes et un appareil d'alimentation en air, l'au moins un orifice de l'écran facial (128) étant raccordé de manière fonctionnelle à l'appareil d'alimentation en air, et l'appareil d'alimentation en air étant configurable pour établir une pression d'air à l'intérieur de la zone respiratoire fermée supérieure à une pression d'air du milieu ambiant.

12. Ensemble de protection individuelle (200) comprenant :
un masque facial (210), un casque, ou une cagoule comportant une visière (212) ; et
un respirateur facial de type demi-masque (120) selon l'une quelconque des revendications 1 à 10, le respirateur facial de type demi-masque (120) étant adapté à être agencé entre le masque facial, le casque, ou la cagoule et le visage de l'utilisateur.
